# DEMANDE DE BREVET EUROPEEN

(11) **EP 1 435 228 A1**
(43) Date de publication de la demande: **07.07.2004**
(21) Numéro de dépôt: 03104984.4
(22) Date de dépôt: 26.12.2003
(51) Int. Cl.: A61K 7/13

(54) **Composition de teinture des fibres kératiniques comprenant un colorant direct trihétéroylméthane ou un précurseur leuco de celui-ci et procédé de teinture la mettant en oeuvre**

(30) Priorité: 30.12.2002 FR 0216849
(71) Demandeur: L'OREAL, 75008 Paris (FR)
(72) Inventeur: Guerin, Frédéric, 75009 Paris (FR); Lagrange, Alain, 77700 Coupvray (FR)
(74) Mandataire: Poulin, Gérard

(57) **Abrégé**

Composition de teinture des fibres kératiniques, en particulier des fibres kératiniques humaines et, plus particulièrement, des cheveux, qui comprend au moins un composé choisi parmi les colorants directs de type hétéroylméthane, plus précisément trihétéroylméthane et les précurseurs leuco de ceux-ci.

L'invention concerne, en outre, un procédé de teinture des fibres kératiniques, en particulier des fibres kératiniques humaines et, plus particulièrement, des cheveux qui met en oeuvre ladite composition.

## Description

La présente invention concerne une composition de teinture des fibres kératiniques, en particulier des fibres kératiniques humaines et, plus particulièrement, des cheveux, qui comprend au moins un composé choisi parmi les colorants directs de type hétéroylméthane, plus précisément trihétéroylméthane et les précurseurs leuco de ceux-ci.

L'invention concerne, en outre, un procédé de teinture des fibres kératiniques, en particulier des fibres kératiniques humaines et, plus particulièrement, des cheveux qui met en oeuvre ladite composition.

Il existe de nombreuses compositions et de nombreux procédés pour teindre les fibres kératiniques et, en particulier, les cheveux humains.

Ainsi, il est connu de teindre les fibres kératiniques et, en particulier, les cheveux humains avec des compositions tinctoriales contenant des précurseurs de colorant d'oxydation, en particulier des ortho ou paraphénylènediamines, des ortho ou paraaminophénols, des composés hétérocycliques, tels que des dérivés de diaminopyrazole, appelés généralement « bases d'oxydation ». Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu, d'une part, au niveau des bases d'oxydation et, d'autre part, au niveau des coupleurs, permet l'obtention d'une riche palette de couleurs.

La coloration, dite « permanente », obtenue grâce à ces colorants d'oxydation, doit, par ailleurs, satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, l'ondulation permanente, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles, tout au long d'une même fibre kératinique, qui peut être en effet différemment sensibilisée, c'est-à-dire abîmée entre sa pointe et sa racine.

Il s'avère, en conclusion, que si les associations base-coupleur classiques permettent d'obtenir une palette étendue de couleurs, elles ne permettent pas de satisfaire aux critères énumérés plus haut et, notamment, conduisent souvent à la génération dans la fibre de produits de couplage variés et mal définis, ce qui pose des problèmes d'innocuité et/ou des problèmes de ténacité difficiles à maîtriser, comme par exemple un virage sélectif.

Une autre manière de teindre les fibres kératiniques, en particulier les cheveux, est d'utiliser des colorants directs.

Les colorants classiques qui sont utilisés sont en particulier des colorants du type nitrés benzèniques, anthraquinoniques, nitropyridiniques, azoïques, azoïques cationiques, xanthéniques, acridiniques aziniques, triarylméthanes, benzéniques nitrés ou des colorants naturels.

Ces colorants qui sont des molécules colorées et colorantes ayant une affinité pour les fibres sont appliqués sur les fibres kératiniques pendant un temps nécessaire à l'obtention de la coloration désirée, puis rincés.

L'utilisation des colorants directs est de ce fait très répandue car ils présentent, en outre, certains avantages par rapport aux précurseurs de colorants d'oxydation et, notamment, une diminution des risques potentiels d'allergie et l'absence de sensibilisation du cheveu due au processus oxydatif.

Les colorations obtenues sont cependant temporaires ou semi-permanentes, car la nature des interactions qui lient les colorants directs à la fibre kératinique, et leur désorption de la surface et/ou du coeur de la fibre sont responsables de leur faible puissance tinctoriale et de leur mauvaise tenue aux lavages, aux intempéries, ou à la transpiration. Ces colorants directs sont en outre généralement sensibles à la lumière du fait de la faible résistance du chromophore vis-à-vis des attaques photochimiques et conduisent dans le temps à un affadissement de la coloration des cheveux.

Il existe donc un besoin pour une composition de teinture des fibres kératiniques, en particulier des fibres kératiniques humaines et, plus particulièrement, des cheveux, qui soit d'une innocuité, d'une compatibilité, totale, vis-à-vis des fibres kératiniques, qui soit peu sélective, qui donne une grande variété de couleurs, puissantes, et qui permette, en outre, d'obtenir une coloration des fibres kératiniques tenace, stable, résistante aux agents extérieurs, tels que la lumière, les intempéries, le lavage, la transpiration, et les frottements et aux traitements ultérieurs, tels que l'ondulation permanente.

Il existe encore un besoin pour une composition de teinture qui permette le traitement de toutes sortes de fibres kératiniques, de tous types de cheveux, par exemple des cheveux blancs, même si ces cheveux ont subi préalablement un traitement, tel qu'un traitement de décoloration ou d'ondulation permanente. Il existe enfin un besoin pour une composition qui réalise la teinture sans qu'il soit nécessaire de réaliser, au préalable, une sensibilisation de la fibre kératinique, du cheveu.

Le but de la présente invention est de fournir une composition de teinture des fibres kératiniques, en particulier des fibres kératiniques humaines et, plus particulièrement, des cheveux qui réponde, entre autres, aux besoins énumérés ci-dessus.

Le but de la présente invention est encore de fournir une composition de teinture des fibres kératiniques qui ne présente pas les inconvénients, défauts, limitations et désavantages des compositions de teinture de l'art antérieur, qu'il s'agisse des compositions de teinture mettant en oeuvre une association base-coupleur, ou des compositions mettant en oeuvre un colorant direct.

Ce but et d'autres encore sont atteints, conformément à l'invention, par une composition pour la teinture des fibres kératiniques, comprenant, dans un milieu cosmétique approprié pour la teinture, au moins un colorant choisi parmi les composés répondant à la formule (I) ou (II) suivante ou leurs formes tautomères, et leurs sels d'addition : 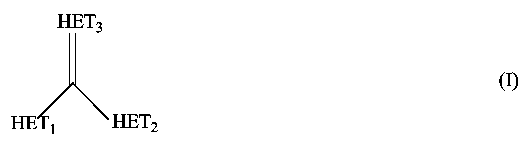 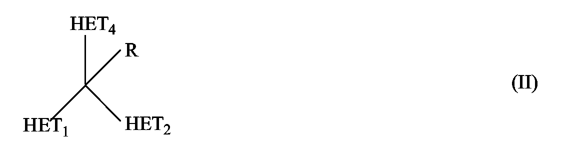 dans lesquelles :
- R représente un atome d'hydrogène ou d'halogène ; un radical hydroxyle ; un radical alcoxy ; ou un radical alkylthio ;
- HET₁, HET₂, HET₃ et HET₄ qui peuvent être identiques ou différents représentent chacun indépendamment un hétérocycle.

Dans la formule (II) ci-dessus, le terme alkyle utilisé pour les radicaux alkylthio, ainsi que pour les groupements comportant une partie alkyle, signifie, sauf indication différente, une chaîne carbonée, linéaire ou ramifiée, comportant de 1 à 30 atomes de carbone, de préférence de 1 à 8, mieux de 1 à 4, pouvant être portée et/ou interrompue par un ou plusieurs atomes d'oxygène de soufre ou d'azote, pouvant être substituée par un ou plusieurs groupes choisis parmi les atomes d'halogène, tel le chlore, le brome, l'iode et le fluor ; les hétérocycles ; les radicaux aryle ; hydroxyle ; alcoxy ; amino ; acyle ; carboxamido ; -CO₂H ; -SO₃H ; -PO₃H₂ ; -PO₄H₂ ; -NHSO₃H ; sulfonamide ; monoalkylamino ; trialkylammonium ; ou bien encore par un radical dialkylamino, dans lequel les deux groupements alkyle peuvent former, conjointement avec l'atome d'azote dudit groupement dialkyl(C₁-C₄)amino, auquel ils sont liés, un cycle pouvant être interrompu par un ou plusieurs atomes d'azote, d'oxygène ou de soufre.

De même, selon l'invention, le terme alcoxy utilisé pour les radicaux alcoxy signifie, sauf indication différente, une chaîne O-alkyle, le terme alkyle ayant la signification indiquée ci-dessus. Les groupes acyle ont de préférence de 2 à 4 atomes de carbone.

Selon l'invention, on entend par hétérocycle, un cycle aromatique ou non contenant 5, 6 ou 7 sommets, et de 1 à 3 hétéroatomes choisis parmi les atomes d'azote, de soufre et d'oxygène. Ces hétérocycles peuvent être condensés sur d'autres hétérocycles, ou sur d'autr es cycles notamment aromatiques tels qu'un groupement phényle. Ils peuvent être substitués par un atome d'halogène ; un radical alkyle ; un radical alcoxy ; un radical hydroxyle ; un radical amino ; un radical alkylamino ; dialkylamino, dans lequel les deux groupements alkyle peuvent conjointement avec l'atome d'azote, auquel il sont liés, former un cycle pouvant être interrompu par un ou plusieurs atomes d'azote, d'oxygène ou de soufre. Ces hétérocycles peuvent, en outre, être quaternisés par un radical alkyle. Les termes alkyle et alcoxy ont les significations indiqués ci-dessus.

Selon une forme de réalisation préférée, les groupes HET₁ à HET₄ représentent un hétérocycle insaturé, de préférence un hétérocycle aromatique.

Parmi les hétérocycles, notamment ceux de HET₁ à HET₄, on peut notamment citer à titre d'exemple les cycles : thiophène, benzothiophène, furane, benzofurane, indole, indoline, carbazole, pyridine, déhydroquinoléine, chromone, julolidine, thiadiazole, triazole, isoxazole, oxazole, thiazole, isothiazole, imidazole, pyrazole, triazine, thiazine, pyrazine, pyridazine, pyrimidine, pyridine, diazépine, oxazépine, benzotriazole, benzoxazole, benzimidazole, benzothiazole, morpholine, pipéridine, pipérazine, azétidine, pyrrolidine, aziridine.

Selon l'invention, on entend par aryle, sauf spécifié autrement, un radical aryle pouvant être substitué par un ou plusieurs radicaux alkyle ; alcoxy ; acyle ; cyano ; carboxamido ; -CO₂H ; -SO₃H ; -PO₃H₂ ; -PO₄H₂ ; hydroxyle ; amino ; monoalkyl(C₁-C₄)amino ; ou dialkyl (C₁-C₄)amino ; dans lequel les deux groupements alkyle peuvent former, conjointement avec l'atome d'azote dudit groupement dialkyl(C₁-C₄)amino, auquel ils sont liés, un cycle pouvant être interrompu par un ou plusieurs atomes d'azote, d'oxygène ou de soufre. De préférence, le groupe aryle est un groupe phényle ou un groupe naphtyle pouvant être substitué comme indiqué ci-dessus.

Les composés (I) peuvent être définis comme étant des colorants directs du type trihétéroylméthane et les composés de formule (II) sont les précurseurs leuco des trihétéroylméthanes de formule (I).

Les composés leuco (II) sont généralement peu ou pas colorés et peuvent être transformés par simple oxydation à l'air ou en présence d'un agent oxydant en un composé trihétéroylméthane de formule (I).

Parmi les composé de formule (I) ou (II) utiles en tant que colorants dans les compositions tinctoriales selon l'invention, on peut notamment citer les composés suivants pour lesquels les contre ions sont ou non spécifiés et leurs sels d'addition :
Chlorure de 1H-Benzo[ij]quinolizinium, 9-[bis(2,3,6,7-tétrahydro-1H,5H-benzo[ij]quinolizin-9-yl)méthylène]-2,3,5,6,7,9-hexahydro-5H-Benzo[a]carbazolium, 11-éthyl-5-[(11-éthyl-11H-benzo[a]carbazol-5-yl)(1-éthyl-1,2,3,4-tétrahydro-5-quinolinyl)méthylène]-Pyrrolo[3,2,1-ij]quinolinium, 8-[bis(1,2,5,6-tétrahydro-4H-pyrrolo[3,2,1-ij]quinolin-8-yl)méthylène]-1,2,4,5,6,8-hexahydro-Tri(9-éthy-9H-carbazol-3-yl)méthane
Bis(6-Chloro-9-éthy-9H-carbazol-3-yl)-(9-éthy-9H-carbazol-3-yl)méthane
Bis(1-(4-sulfo-butyl)-2,3,4,6-tétrahydro-quinolinium)-pyrid-4-yl-méthane
Bis(1-éthyl-2-méthyl-1H-indol-3-yl)-(9-éthy-9H-carbazol-3-yl)méthane
Tri(7-éthyl-7H-benzo[c]carbazol-10-yl)méthane
Bis((6-diméthylamino-3-méthyl-1H-indol-2-yl)- 2-furylméthane
Bis((6-diméthylamino-3-méthyl-1H-indol-2-yl)- (pyrid-4-yl)méthane
Bis(1-éthyl-2-méthyl-1H-indol-3-yl)- 2-thiénylméthane
3-[(1-éthyl-2-méthyl-1H-indol-3-yl)- (9-éthy-9H-carbazol-3-yl) méthylène]-1-éthyl-2-méthyl-3H-indolium
3-[(1-éthyl-2-méthyl-1H-indol-3-yl)-2-thiényl) méthylène]-1-éthyl-2-méthyl-3H-indolium.

Les trihétéroylméthanes de formule (I) et les composés leuco de formule (II) sont des composés connus. Ces composés peuvent être préparés selon des procédés de synthèses bien connus dans la littérature et tels que décrits, par exemple, dans les documents FR-A-2 188 202, US-A-3 995 088, US-A-4 154 463, US-A-4 254 032, US-A-4 355 823, US-A-5 266 699 et US-A-5 362 612.

Il s'avère que les compositions de l'invention permettent, de manière surprenante, d'obtenir des colorations intenses, même sur des cheveux non sensibilisés.

Les compositions selon l'invention permettent d'obtenir des reflets variés chromatiques ou sombres, très puissants, peu sélectifs et tenaces.

Ainsi, les colorants trihétéroyl méthanes de formule (I) ou les leucos de formule (II) inclus dans les compositions de l'invention permettent d'obtenir (après révélation pour les composés leucos (II)) toutes les nuances du vert au bleu, en passant par les rouges et, en particulier, ils permettent d'obtenir aussi les nuances noires.

Les colorations obtenues avec les compositions de l'invention sont tenaces, stables, résistantes, vis-à-vis des intempéries, du lavage, de la transpiration, des frottements et des traitements ultérieurs, tels que l'ondulation permanente.

Ces colorations sont, en particulier, particulièrement tenaces à la lumière.

Le ou les composés de formule (I) ou (II) ci-dessus et/ou leur ou leurs sels d'additions représentent généralement de 0,0001 à 10 % en poids du poids total de la composition de teinture, de préférence de 0,005 à 10 % en poids et, de préférence encore, de 0,01 à 6 % de poids du poids total de la composition de teinture selon l'invention.

La composition tinctoriale conforme à l'invention peut en outre contenir un ou plusieurs colorant(s) direct(s) différent(s) des composés de formule (I) ou (II). Le ou ces colorant (s) direct (s) utile(s) selon l'invention sont par exemple choisis parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs benzéniques, on peut citer de manière non limitative les composés suivants :
- 1,4-diamino-2-nitrobenzène ;
- 1-amino-2 nitro-4-β- hydroxyéthylaminobenzène ;
- 1-amino-2 nitro-4-bis(β-hydroxyéthyl)-aminobenzène ;
- 1,4-Bis(β-hydroxyéthylamino)-2-nitrobenzène ;
- 1-β-hydroxyéthylamino-2-nitro-4-bis-(β-hydroxyéthylamino)-benzène ;
- 1-β-hydroxyéthylamino-2-nitro-4-aminobenzène ;
- 1-β-hydroxyéthylamino-2-nitro-4-(éthyl) (β-hydroxyéthyl)-aminobenzène ;
- 1-amino-3-méthyl-4-β-hydroxyéthylamino-6-nitrobenzène ;
- 1-amino-2-nitro-4-β-hydroxyéthylamino-5-chlorobenzène ;
- 1,2-Diamino-4-nitrobenzène ;
- 1-amino-2-β-hydroxyéthylamino-5-nitrobenzène ;
- 1,2-Bis-(β-hydroxyéthylamino)-4-nitrobenzène ;
- 1-amino-2-tris-(hydroxyméthyl)-méthylamino-5-nitrobenzène ;
- 1-Hydroxy-2-amino-5-nitrobenzène ;
- 1-Hydroxy-2-amino-4-nitrobenzène ;
- 1-Hydroxy-3-nitro-4-aminobenzène ;
- 1-Hydroxy-2-amino-4,6-dinitrobenzène ;
- 1-β-hydroxyéthyloxy-2-β-hydroxyéthylamino-5-nitrobenzène ;
- 1-Méthoxy-2-β-hydroxyéthylamino-5-nitrobenzène ;
- 1-β-hydroxyéthyloxy-3-méthylamino-4-nitrobenzène ;
- 1-β,γ-dihydroxypropyloxy-3-méthylamino-4-nitrobenzène ;
- 1-β-hydroxyéthylamino-4-β,γ-dihydroxypropyloxy-2-nitrobenzène ;
- 1-β,γ-dihydroxypropylamino-4-trifluorométhyl-2-nitrobenzène ;
- 1-β-hydroxyéthylamino-4-trifluorométhyl-2-nitrobenzène ;
- 1-β-hydroxyéthylamino-3-méthyl-2-nitrobenzène ;
- 1-β-aminoéthylamino-5-méthoxy-2-nitrobenzène ;
- 1-Hydroxy-2-chloro-6-éthylamino-4-nitrobenzène ;
- 1-Hydroxy-2-chloro-6-amino-4-nitrobenzène ;
- 1-Hydroxy-6-bis-(β-hydroxyéthyl)-amino-3-nitrobenzène ;
- 1-β-hydroxyéthylamino-2-nitrobenzène ;
- 1-Hydroxy-4-β-hydroxyéthylamino-3-nitrobenzène.

Parmi les colorants directs azoïques, on peut citer les colorants azoïques cationiques décrits dans les demandes de brevets WO 95/15144, WO-95/01772, EP-714954 et WO-A-01/66646 dont le contenu fait partie intégrante de l'invention.

Parmi ces composés, on peut tout particulièrement citer les colorants suivants :
- chlorure de 1,3-diméthyl-2-[[4-(diméthylamino)phényl]azo]-1H-Imidazolium ;
- chlorure de 1,3-diméthyl-2-[(4-aminophényl)azo]-1H-Imidazolium ;
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3^{e} édition :
- Disperse Red 17 ;
- Acid Yellow 9 ;
- Acid Black 1 ;
- Basic Red 22 ;
- Basic Red 76 ;
- Basic Yellow 57 ;
- Basic Brown 16 ;
- Acid Yellow 36 ;
- Acid Orange 7 ;
- Acid Red 33 ;
- Acid Red 35 ;
- Basic Brown 17 ;
- Acid Yellow 23 ;
- Acid Orange 24 ;
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques, on peut citer les colorants suivants :
- Disperse Red 15 ;
- Solvent Violet 13 ;
- Acid Violet 43 ;
- Disperse Violet 1 ;
- Disperse Violet 4 ;
- Disperse Blue 1 ;
- Disperse Violet 8 ;
- Disperse Blue 3 ;
- Disperse Red 11 ;
- Acid Blue 62 ;
- Disperse Blue 7 ;
- Basic Blue 22 ;
- Disperse Violet 15 ;
- Basic Blue 99,
ainsi que les composés suivants :
- 1-N-méthylmorpholiniumpropylamino-4-hydroxyanthraquinone ;
- 1-Aminopropylamino-4-méthylaminoanthraquinone ;
- 1-Aminopropylaminoanthraquinone ;
- 5-β-hydroxyéthyl-1,4-diaminoanthraquinone ;
- 2-Aminoéthylaminoanthraquinone ;
- 1,4-Bis-(β,γ-dihydroxypropylamino)-anthraquinone.

Parmi les colorants aziniques, on peut citer les composés suivants :
- Basic Blue 17 ;
- Basic Red 2.

Parmi les colorants triarylméthaniques, on peut citer les composés suivants :
- Basic Green 1 ;
- Acid blue 9 ;
- Basic Violet 3 ;
- Basic Violet 14 ;
- Basic Blue 7 ;
- Acid Violet 49 ;
- Basic Blue 26 ;
- Acid Blue 7.

Parmi les colorants indoaminiques, on peut citer les composés suivants :
- 2-β-hydroxyéthlyamino-5-[bis-(β-4'-hydroxyéthyl)amino]anilino-1,4-benzoquinone ;
- 2-β-hydroxyéthylamino-5-(2'-méthoxy-4'-amino)anilino-1,4-benzoquinone ;
- 3-N(2'-Chloro-4'-hydroxy)phényl-acétylamino-6-méthoxy-1,4-benzoquinone imine ;
- 3-N(3'-Chloro-4'-méthylamino)phényl-uréido-6-méthyl-1,4-benzoquinone imine ;
- 3-[4'-N-(Ethyl,carbamylméthyl)-amino]phényl-uréido-6-méthyl-1,4-benzoquinone imine.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs additionnels différents des composés de formule (I) ou (II), représentent de préférence de 0,001 à 20% en poids environ du poids total de la composition prête à l'emploi et encore plus préférentiellement de 0,005 à 10% en poids environ.

La composition de la présente invention peut de plus contenir une ou plusieurs base (s) d'oxydation et éventuellement un ou plusieurs coupleur(s) classiquement utilisés pour la coloration par oxydation.

A titre d'exemple de base d'oxydation, on peut citer les paraphénylènediamines, les bis-phénylalkylènediamines, les para-aminophénols, les ortho-aminophénols, les bases hétérocycliques et leurs sels d'addition.

Les coupleurs sont par exemple les coupleurs métaphénylènediamines, les coupleurs méta-aminophénols, les coupleurs métadiphénols, les coupleurs naphtaléniques, les coupleurs hétérocycliques et leurs sels d'addition.

Lorsqu'ils sont présents, le ou les base(s) d'oxydation et/ou le ou les coupleurs sont chacun généralement présents en une quantité comprise entre 0,001 et 10 % en poids environ du poids total de la composition tinctoriale, de préférence entre 0,005 et 6 %.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci-dessus sont en général présents en quantité comprise pour chacun d'eux entre 0,01 et 20 % en poids par rapport au poids de la composition.

De préférence, les compositions contiennent un solvant choisi parmi les alcanols en C₂-C₄ et les polyols de poids moléculaire inférieur à 1 000.

De préférence, des compositions contiennent au moins un agent tensioactif et/ou au moins un agent épaississant minéral ou organique.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, de préférence entre 5 et 11 environ, de préférence encore de 6 à 8,5.

Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 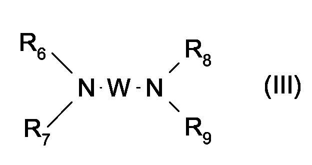 dans laquelle W est un reste propylène éventuellement substitué par un groupement hydroxyle ou un radical alkyle en C₁-C₄ ; R₆, R₇, R₈ et R₉, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a aussi pour objet un procédé de teinture directe des fibres kératiniques et, en particulier, des fibres kératiniques humaines, telles que les cheveux, qui comprend l'application d'au moins une composition tinctoriale contenant au moins un composé, colorant, de formule (I) ou (II) telle que définie précédemment sur les fibres kératiniques. Après un temps de pause, les fibres kératiniques sont rincées laissant apparaître des fibres colorées. Le temps de pause est généralement compris entre 3 à 50 minutes environ, de préférence 5 à 30 minutes environ.

Rappelons que par coloration ou teinture directe, on entend une coloration effectuée sans agent oxydant autre que l'oxygène de l'air.

Lorsque la composition tinctoriale comprend soit au moins un composé de formule (II), soit au moins un composé de formule (I), et au moins une base d'oxydation, et éventuellement un ou plusieurs coupleur(s), la composition tinctoriale contient de préférence un oxydant.

L'invention a donc également pour objet un procédé de teinture des fibres kératiniques et, en particulier, des fibres kératiniques humaines, telles que les cheveux, dans lequel on applique sur lesdites fibres au moins une composition de teinture comprenant soit au moins un composé de formule (II), soit au moins un composé de formule (I) et au moins une base d'oxydation et éventuellement un ou plusieurs coupleurs, la couleur étant révélée à pH acide, neutre ou alcalin, à l'aide d'un agent oxydant.

Les agents oxydants utilisables sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

L'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention. La composition oxydante peut également renfermer divers adjuvants utilisés classiquement dans les compositions pour la teinture des cheveux et tels que définis précédemment.

En cas de mélange avec la composition tinctoriale, le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11, mieux entre 6 et 8,5. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Un autre objet de l'invention est un dispositif à plusieurs compartiments ou « kit » de teinture, dans lequel un premier compartiment renferme une composition tinctoriale définie ci-dessus, comprenant soit au moins un composé de formule (II), soit au moins un composé de formule (I) et au moins une base d'oxydation et éventuellement un ou plusieurs coupleur(s), et un deuxième compartiment renferme la composition oxydante.

Ce dispositif peut être équipé d'un moyen permettant de délivrer sur les cheveux le mélange souhaité, tel que les dispositifs décrits dans le brevet FR-2 586 913 au nom de la demanderesse.

Dans le cas d'une composition contenant au moins un composé de formule (II), il est souhaitable d'éviter le contact entre lesdits composés et l'oxygène de l'air. Un mode de conditionnement particulièrement adapté peut être alors un dispositif aérosol.

Avec les colorants de l'invention, on peut également réaliser de la coloration directe éclaircissante en l'absence de colorants d'oxydation et en présence d'un agent oxydant dans des conditions telles (par exemple peroxyde d'hydrogène en milieu alcalin) que celui-ci soit apte à éclaircir les fibres kératiniques par action sur les pigments initialement présents dans lesdites fibres.

Les exemples qui suivent servent à illustrer l'invention sans toutefois présenter un caractère limitatif.

### Exemple 1

On a préparé la composition de teinture suivante :

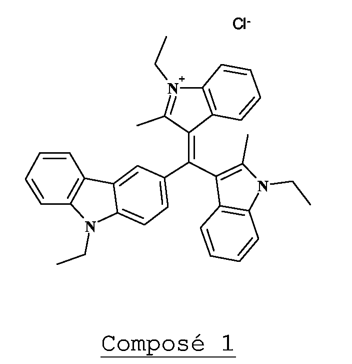

| | |
|---|---|
| 3-[(1-éthyl-2-méthyl-1H-indol-3-yl)- (9-éthyl-9H-carbazol-3-yl) méthylène]-1-éthyl-2-méthyl-3H-indolium chlorure | 0,56 g |
| Alcool benzylique | 4,0 g |
| Polyéthylèneglycol 6OE | 6,0 g |
| Hydroxyéthylcellulose | 0,7 g |
| Alkylpolyglucoside en solution aqueuse à 60% M.A* | 4,5 g M.A |
| Tampon phosphate q.s. pH | 7 |
| Eau déminéralisée q.s.p. | 100, g |

| | |
|---|---|
| * Matière Active | |

On a appliqué la composition ci-dessus sur des mèches de cheveux gris naturels ou permanentés à 90% de blancs et on a laissé poser pendant 20 minutes. Après rinçage à l'eau courante et séchage, les cheveux ont été teints dans une nuance rouge.

### Exemple 2

On a préparé la composition de teinture suivante :

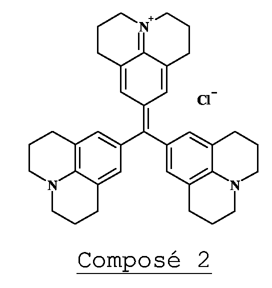

| | |
|---|---|
| Chlorure de 1H-Benzo[ij]quinolizinium, 9-[bis(2,3,6,7-tetrahydro-1H,5H-benzo[ij]quinolizin-9-yl)méthylène]-2, 3, 5, 6, 7, 9-hexahydro- (10⁻³ mole) | 0, 55 g |
| Diéthanolamide oléïque | 3 g |
| Acide laurique | 1 g |
| Monoéthyléther de l'éthylèneglycol | 5 g |
| Hydroxyéthylcellulose | 2 g |
| 2-amino-2-méthyl-1-propanol q.s. pH | 9,5 |
| Eau déminéralisée q.s.p. | 100 g |

On a appliqué la composition ci-dessus sur des mèches de cheveux gris naturels ou permanentés à 90% de blancs et on a laissé poser pendant 30 minutes. Après rinçage à l'eau courante et séchage, les cheveux ont été teints dans une nuance bleu.

### Exemples 3 à 6

On a préparé les cinq compositions de teinture directe réunies dans le tableau suivant :

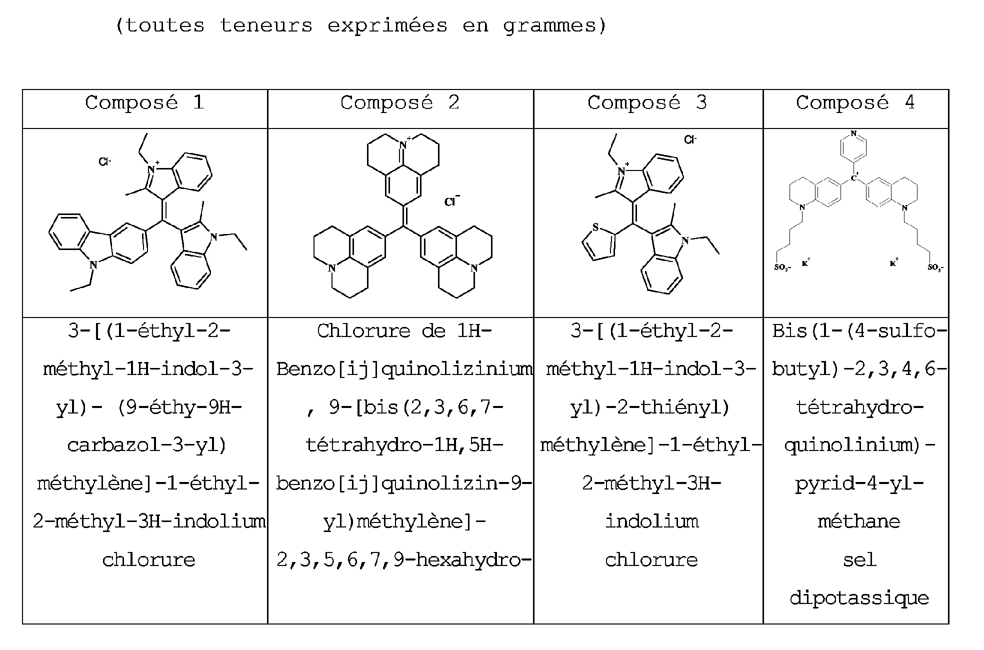

| | Exemple 3 | Exemple 4 | Exemple 5 | Exemple 6 |
|---|---|---|---|---|
| Colorant direct de formule (1) | 0,2 | | | |
| Colorant direct de formule (2) | | 0,2 | | |
| Colorant direct de formule (3) | | | 0,2 | |
| Colorant direct de formule (4) | | | | 0,2 |
| Acide polyacrylique réticulé vendu sous la dénomination Carbopol 2984 par la société Goodrich | 1,0 MA* | | | |
| Copolymère acrylate d'ammonium/acrylamide vendu sous la dénomination Bozepol C Nouveau par la société Hoechst | | 1,0 MA* | | |
| Copolymère acide méthacrylique/acrylate d'éthyle réticulé vendu en dispersion aqueuse à 38% de matière active sous la dénomination Viscoatex 538C par la société Coatex | | | 1,0 MA* | |
| Copolymère acide acrylique/acrylate d'éthyle réticulé vendu en dispersion aqueuse à 28% de matière active sous la dénomination Aculyn 33 par la société Rohm & Haas | | | | 1,0 MA* |
| Ethanol | 10 | 10 | 10 | 10 |
| 2-amino-2-méthyl-1-propanol qs | pH 9 | pH 9 | pH 9 | pH 9 |
| Eau déminéralisée qsp | 100 | 100 | 100 | 100 |

| | | | | |
|---|---|---|---|---|
| MA* désigne Matière Active | | | | |

Les compositions ci-dessus ont été appliquées chacune pendant 30 minutes sur des mèches de cheveux gris naturels à 90 % de blancs. Les mèches de cheveux ont ensuite été rincées, lavées avec un shampooing standard puis séchées.

Les mèches ont été teintes dans les nuances suivantes :

| Exemples | Nuances obtenues |
|---|---|
| 3 | Rouge puissant |
| 4 | Bleu puissant |
| 5 | Orange puissant |
| 6 | bleu puissant |

### Exemples 7 à 10

On a préparé les compositions 7 (A) à 10 (A), conformes à l'invention, suivantes (teneurs en grammes) :

| COMPOSITION | 7 (A) | 8 (A) | 9 (A) | 10 (A) |
|---|---|---|---|---|
| Paratoluylènediamine | 0,25 | - | - | - |
| Para-aminophénol | 0,30 | 0,50 | 0,15 | - |
| Paraphénylènediamine | - | 0,20 | | 0,30 |
| 5-N- (β-hydroxyéthyl)amino 2-méthyl phénol | 0,5 | 0,8 | 0,17 | - |
| 5-amino 2-méthyl phénol | - | - | - | 0,30 |
| Colorant de structure (2) | 0,15 | - | - | - |
| Colorant de structure (3) | - | 0,20 | 0,05 | - |
| Colorant de structure (4) | - | - | - | 0,1 |
| Support de teinture commun(*) | (*) | (*) | (*) | (*) |
| Eau q.s.p. | 100 g | 100 g | 100 g | 100 g |

| | | | | |
|---|---|---|---|---|
| (*) support de teinture commun : - Alcool oléique polyglycérolé à 2 moles de glycérol 4,0 g - Alcool oléique polyglycérolé à 4 moles de glycérol, à 78 % de matières actives (M.A.) 5,69 g M.A. - Acide oléique 3,0 g - Amine oléique à 2 moles d'oxyde d'éthylène vendue sous la dénomination commerciale ETHOMEEN O12 par la société AKZO 7,0 g - Laurylamino succinamate de diéthylaminopropyle, sel de sodium, à 55 % de M.A. 3,0 g M.A. - Alcool oléique 5,0 g - Diéthanolamide d'acide oléique 12,0 g - Propylèneglycol 3,5 g - Alcool éthylique 7,0 g - Dipropylèneglycol 0,5 g - Monométhyléther de propylèneglycol 9,0 g - Métabisulfite de sodium en solution aqueuse, à 35 % de M.A. 0,455 g M.A. - Acétate d'ammonium 0,8 g - Antioxydant, séquestrant q.s. - Parfum, conservateur q.s. - Ammoniaque à 20 % de NH₃ 10,0 g | | | | |

Au moment de l'emploi, on a mélangé chacune de ces compositions 7 (A) à 10 (A) avec une quantité égale d'une composition (B) constituée par une solution de peroxyde d'hydrogène à 20 volumes (6 % en poids).

Chaque composition résultante (composition prête à l'emploi conforme à l'invention) a été appliquée pendant 30 minutes sur des mèches de cheveux gris naturels à 90 % de blancs. Les mèches de cheveux ont ensuite été rincées, lavées avec un shampooing standard puis séchées.

Les mèches de cheveux ont été teintes dans les nuances figurant dans le tableau ci-dessous :

| EXEMPLE [COMPOSITION] | NUANCE OBTENUE |
|---|---|
| 7 [7 (A)] | Blond foncé à reflet bleu |
| 8 [8 (A)] | Blond à reflet orange |
| 9 [9 (A)] | Blond clair à reflet orange |
| 10 [10 (A)] | Blond à reflet bleu |

Les nuances obtenues ont présenté une très bonne ténacité aux shampooings ultérieurs.

Selon une variante de l'invention, les colorants directs de structures (2), (3) et (4) peuvent être incorporés dans les compositions colorantes 7 (A), 8 (A), 9 (A) et 10 (A) au moment de l'emploi.

### Exemple 11

On a préparé la composition 11 (A) suivante :
- 1,4-diamino benzène 0,40 g
- 5-amino 2-méthyl phénol 0,45 g
- Support de teinture commun tel que décrit précédemment pour les exemples 7 à 10 (*)
- Eau déminéralisée q.s.p. 100 g

On a préparé la composition 11 (A') suivante : 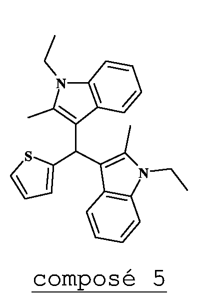
- Colorant cationique Bis(1-éthyl-2-méthyl-1H-indol-3-yl)- 2-thiènylméthane 4 g
- Polyammonium quaternaire vendu sous la dénomination commerciale CELQUAT SC-240 par la société National Starch 10 g
- Sciure de bois q.s.p. 100 g

Au moment de l'emploi, on a mélangé une partie en poids de la composition 11 (A) ci-dessus avec 0,1 partie en poids de la composition 11 (A') et avec une partie en poids d'une composition (B) constituée par une solution de peroxyde d'hydrogène à 20 volumes (6 % en poids).

La composition résultante a été appliquée pendant 30 minutes sur des mèches de cheveux gris naturels à 90 % de blancs. Les cheveux ont ensuite été rincées, lavées avec un shampooing standard puis séchées.

Les cheveux ont été teints dans une nuance châtain clair à reflet orange résistant très bien aux shampooings ultérieurs.

## Revendications

1. Composition pour la teinture des fibres kératiniques, comprenant, dans un milieu cosmétique approprié pour la teinture, au moins un colorant choisi parmi les composés répondant à la formule (I) ou (II) suivante ou leurs formes tautomères, et leurs sels d'addition : 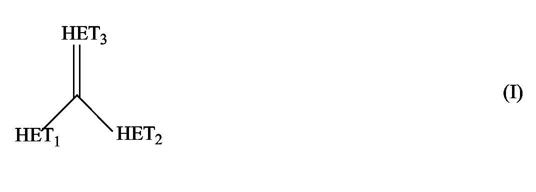 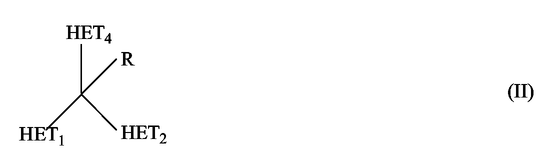 dans lesquelles :
- R représente un atome d'hydrogène ou d'halogène ; un radical hydroxyle ; un radical alcoxy ; ou un radical alkylthio ;
- HET₁, HET₂, HET₃ et HET₄ qui peuvent être identiques ou différents représentent chacun indépendamment un hétérocycle.

2. Composition de teinture selon la revendication 1, **caractérisé en ce que** les groupes HET₁ à HET₄ représentent un hétérocycle insaturé.

3. Composition selon la revendication 2, dans laquelle les groupes HET₁ à HET₄ représentent un hétérocycle aromatique.

4. Composition selon l'une quelconque des revendications 1 à 3, dans laquelle les hétérocycles, et notamment ceux de HET₁ à HET₄, sont choisis parmi les hétérocycles suivants : thiophène, benzothiophène, furane, benzofurane, indole, indoline, carbazole, pyridine, déhydroquinoléine, chromone, julolidine, thiadiazole, triazole, isoxazole, oxazole, thiazole, isothiazole, imidazole, pyrazole, triazine, thiazine, pyrazine, pyridazine, pyrimidine, pyridine, diazépine, oxazépine, benzotriazole, benzoxazole, benzimidazole, benzothiazole, morpholine, pipéridine, pipérazine, azétidine, pyrrolidine, aziridine.

5. Composition de teinture selon l'une quelconque des revendications 1 à 4, **caractérisée en ce qu'**elle comprend un composé de formule (I) ou (II) choisi parmi les composés suivants pour lesquels les contre ions sont ou non spécifiés et leurs sels d'addition :
Chlorure de 1H-Benzo[ij]quinolizinium, 9-[bis(2,3,6,7-tétrahydro-1H,5H-benzo[ij]quinolizin-9-yl)méthylène]-2,3,5,6,7,9-hexahydro-5H-Benzo[a]carbazolium, 11-éthyl-5-[(11-éthyl-11H-benzo[a]carbazol-5-yl)(1-éthyl-1,2,3,4-tétrahydro-5-quinolinyl)méthylène]-Pyrrolo[3,2,1-ij]quinolinium, 8-[bis(1,2,5,6-tétrahydro-4H-pyrrolo[3,2,1-ij]quinolin-8-yl)méthylène]-1,2,4,5,6,8-hexahydro-Tri(9-éthy-9H-carbazol-3-yl)méthane
Bis(6-Chloro-9-éthy-9H-carbazol-3-yl)-(9-éthy-9H-carbazol-3-yl)méthane
Bis(1-(4-sulfo-butyl)-2,3,4,6-tétrahydro-quinolinium)-pyrid-4-yl-méthane
Bis(1-éthyl-2-méthyl-1H-indol-3-yl)-(9-éthy-9H-carbazol-3-yl)méthane
Tri(7-éthyl-7H-benzo[c]carbazol-10-yl)méthane
Bis((6-diméthylamino-3-méthyl-1H-indol-2-yl)- 2-furylméthane
Bis((6-diméthylamino-3-méthyl-1H-indol-2-yl)- (pyrid-4-yl)méthane
Bis(1-éthyl-2-méthyl-1H-indol-3-yl)- 2-thiénylméthane
3-[(1-éthyl-2-méthyl-1H-indol-3-yl)-(9-éthy-9H-carbazol-3-yl)méthylène]-1-éthyl-2-méthyl-3H-indolium
3-[(1-éthyl-2-méthyl-1H-indol-3-yl)-2-thiényl) méthylène]-1-éthyl-2-méthyl-3H-indolium.

6. Composition selon l'une quelconque des revendications précédentes, **caractérisée en ce que** le ou les composés de formule (I) ou (II) et/ou le ou les sel(s) d'addition représentent de 0,0001 à 10 % en poids du poids total de la composition de teinture, de préférence de 0,005 à 10 % en poids et, de préférence encore, de 0,01 à 6 % en poids du poids total de la composition de teinture.

7. Composition selon l'une quelconque des revendications 1 à 6, **caractérisée en ce qu'**elle contient, en outre, un ou plusieurs colorant(s) direct(s) différent(s) des composés de formule (I) ou (II) .

8. Composition selon la revendication 7, **caractérisée en ce que** le ou les colorant(s) direct(s) différent(s) des composés de formule (I) ou (II) sont choisis parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

9. Composition selon l'une quelconque des revendications 7 et 8, **caractérisée en ce que** le ou les colorant(s) direct(s) différents des composés de formule (I) ou (II) représentent de 0,001 à 20 % en poids, de préférence de 0,005 à 10 % en poids du poids total de la composition de teinture.

10. Composition selon l'une quelconque des revendications précédentes, **caractérisée en ce qu'**elle contient, en outre, une ou plusieurs base(s) d'oxydation et éventuellement un ou plusieurs coupleur(s).

11. Composition selon la revendication 10, **caractérisé en ce que** la ou les base(s) d'oxydation sont choisies parmi les paraphénylènediamines, les bis-phénylalkylènediamines, les para-aminophénols, les ortho-aminophénols, les bases hétérocycliques et leurs sels d'addition.

12. Composition selon l'une quelconque des revendications 10 et 11, **caractérisée en ce que** le ou les coupleur(s) sont choisi(s) parmi les coupleurs métaphénylènediamines, les coupleurs méta-aminophénols, les coupleurs métadiphénols, les coupleurs naphtaléniques, les coupleurs hétérocycliques et leurs sels d'addition.

13. Composition selon l'une quelconque des revendications 10 à 12, **caractérisée en ce que** le ou les base(s) d'oxydation et/ou le ou les coupleur(s) sont présents chacun en une quantité de 0,001 à 10 % en poids, de préférence de 0,005 à 6 % en poids du poids total de la composition de teinture.

14. Composition selon l'une quelconque des revendications précédentes, **caractérisée en ce qu'**elle a un pH de 3 à 12, de préférence de 5 à 11, de préférence encore de 6 à 8,5.

15. Procédé de teinture directe des fibres kératiniques et, en particulier, des fibres kératiniques humaines, telles que les cheveux, **caractérisé en ce qu'**il comprend l'application d'au moins une composition de teinture selon l'une quelconque des revendications 1 à 14 contenant au moins un composé de formule (I) ou (II) sur les fibres kératiniques, puis l'observation d'un temps de pause suivi du rinçage des fibres.

16. Procédé selon la revendication 15, **caractérisé en ce que** le temps de pause est de 3 à 50 minutes, de préférence de 5 à 30 minutes.

17. Procédé de teinture des fibres kératiniques et, en particulier, des fibres kératiniques humaines, telles que les cheveux, dans lequel on applique sur lesdites fibres au moins une composition de teinture comprenant soit au moins un composé de formule (II), soit au moins un composé de formule (I) et au moins une base d'oxydation et éventuellement un ou plusieurs coupleurs, la couleur était révélée à pH acide, neutre ou alcalin, à l'aide d'un agent oxydant.

18. Procédé selon la revendication 17, **caractérisé en ce que** l'agent oxydant est choisi parmi le peroxyde d'hydrogène, le peroxyde d'urée, les bromates de métaux alcalins, les persels tels que les perborates et persulfates, les peracides et les enzymes oxydases, tel que les peroxydases, les oxydo-réducteurs à 2 électrons, comme les unicases et les oxygénases à 4 électrons, comme les laccases.

19. Dispositif à plusieurs compartiments ou « kit » de teinture, **caractérisé en ce qu'**il comprend un premier compartiment renfermant une composition tinctoriale selon l'une quelconque des revendications 1 à 14, comprenant soit au moins un composé de formule (II), soit au moins un composé de formule (I) et au moins une base d'oxydation et éventuellement un ou plusieurs coupleurs, et un deuxième compartiment renfermant une composition oxydante.
